# EUROPEAN PATENT APPLICATION

(11) **EP 0 901 778 A2**
(43) Date of publication of application: **17.03.1999**
(21) Application number: 98305906.4
(22) Date of filing: 24.07.1998
(51) Int. Cl.: A61F 5/44, A61B 5/20

(54) **Urine meter assemblies**

(30) Priority: 13.08.1997 GB 9717139
(71) Applicant: Smiths Industries Public Limited Company, London, NW11 8DS (GB)
(72) Inventor: Batchelor, Kester Julian, Burnham-on-Sea, Somerset, TA8 2RY (GB)
(74) Representative: Flint, Jonathan McNeill

(57) **Abstract**

A urine meter assembly 3 is connected to the inlet 20 of a flexible urine bag 2 and to the outlet end of a urine tube 1. The assembly has a transparent chamber 31 with a filter 36 and drip outlet 35 at its upper end. An optical drip sensor 40 is removably fitted onto the chamber 31 to detect drips from the outlet. A counter 43, 44 counts the drips and displays an indication of the volume of urine having flowed through the assembly, the counter being switchable to display the volume collected over different times.

## Description

This invention relates to urine meter assemblies of the kind including a urine receptacle, a urine tube extending from the patient to the urine receptacle and a urine meter including a chamber connected intermediate the receptacle and the tube.

Following surgery, it is often necessary for a patient to be connected by a urinary catheter to a urine drainage bag, so that discharged urine can be collected for disposal. The bag may be graduated to give an approximate measure of the contents. Where a more accurate measure of discharged urine is needed, a urine bag assembly is used including a urine meter in the form of a rigid, graduated container with an inlet connected to receive the discharged urine. The volume of the meter is less than that of the bag so it is necessary periodically to empty the contents of the meter into the bag. The meter may be mounted above the bag, such as described, for example, in WO 89/05119, WO 95/13016 or US 4579126. In such arrangements, the meter has an outlet with a tap at its lower end, which is opened to allow urine to drain from the meter to the bag. Alternatively, the meter may hang alongside the front of the bag, such as described, for example in GB2073595, GB 2191702, EP 471413, US 4699155, US 4301813 or US 4305405. In the latter arrangement, when the meter needs emptying, it is lifted above the bag and tipped so that its contents drain through an outlet into the bag. It is a disadvantage to have to empty the contents of the meter repeatedly. Also, many forms of conventional urine meter are not suitable for general use with any urine receptacle but are integrally combined with a bag or other receptacle forming a urine meter assembly.

It is an object of the present invention to provide an improved urine meter assembly.

According to the present invention there is provided a urine meter assembly of the above-specified kind, characterised in that the meter includes drip outlet at an upper end of the chamber and connected to receive urine discharged from the tube, and an outlet at a lower end of the chamber coupled with an inlet to the urine receptacle, and that the meter includes a drip sensor for detecting drips from the drip outlet and a counter for counting the drips and for providing an indication of the quantity of urine having flowed through the meter assembly.

The drip sensor is preferably removable from the chamber and may include an optical detector including a light source located on one side of the chamber and a light detector located on the opposite side of the chamber. The meter is preferably self supporting. The meter preferably includes a filter upstream of the drip outlet. The outlet at the lower end of the chamber may have a restriction in a lumen through the outlet. The chamber preferably has a male coupling at its upper and at its lower end. The urine receptacle may be a flexible bag. The counter is preferably switchable to display an indication of the quantity collected over different times.

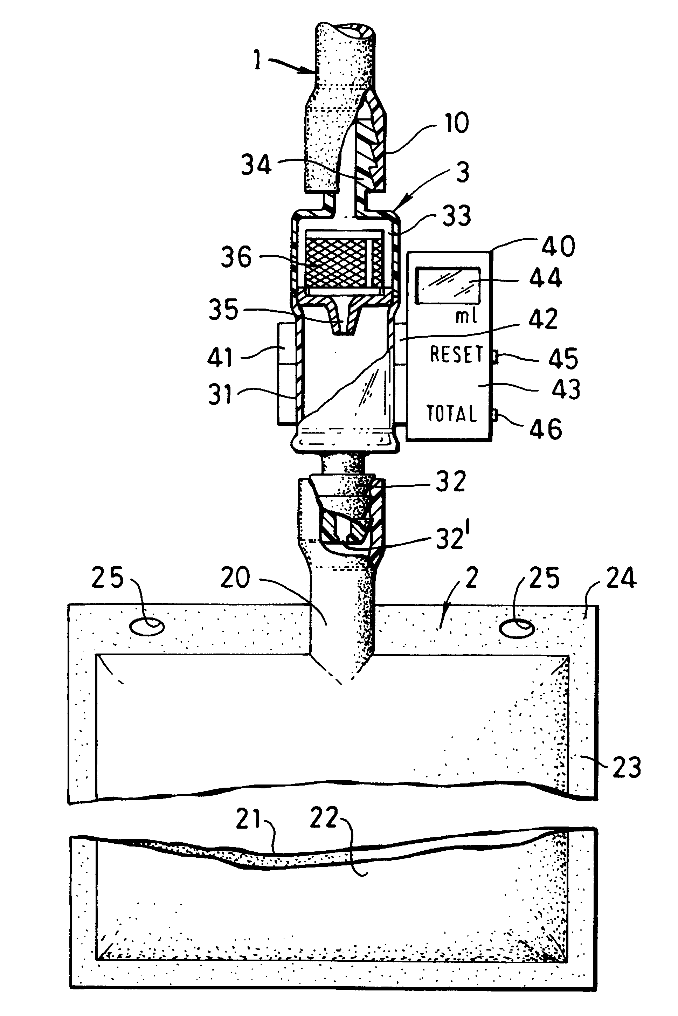

A urine meter assembly according to the present invention, will now be described, by way of example, with reference to the accompanying drawing, which shows the assembly schematically.

The assembly includes a conventional urine tube 1, the patient end of which (not shown) is arranged to receive urine discharged by the patient. The patient end of the tube 1 may be connected to a urethral catheter or to a penile sheath. The machine end 10 of the tube 1 is connected to a urine bag 2, or other urine receptacle, via a urine meter 3. The bag 2 has a short inlet tube 20 at its upper end, which may be cut down from a longer length. The bag 2 is of a conventional kind, comprising two flexible, transparent walls 21 and 22 welded together around their outer edge 23. The upper edge 24 of the bag has mounting apertures 25 or other formations by which the bag can be hung from a stand or the side of a bed.

The meter 3 has a vertically-oriented, transparent, tubular chamber 31 with an outlet coupling 32, which is a push fit in the inlet tube 20 of the urine bag 2. The outlet coupling 32 has a lumen restriction 32' to limit the maximum rate of flow through the chamber 31. At its upper end, the chamber 31 has an inlet assembly 33 comprising a male coupling 34 on which the machine end 10 of the urine tube 1 is push fitted. The lower end of the inlet assembly 33 is formed with a short spigot 35 projecting down a short distance inside the chamber 31. Internally, the spigot 35 is reduced in diameter to form a drip outlet for urine flowing through the inlet assembly 33, that is, the diameter is such that the urine can only flow as separate drips, not as a continuous stream, regardless of the flow rate. The restricted outlet 32' also helps ensure that the flow from the drip spigot 35 is in the form of drops rather than a continuous stream. Between the drip outlet spigot 35 and the inlet coupling 34, the inlet assembly 33 has an in-line basket type mesh filter 36 with a mesh size of about 200µm. The filter 36 collects blood clots, string and other solid matter larger than a certain size and thereby prevents them passing to the drip outlet 35, where they could cause blockage. The filter 36 is visible through the wall of the chamber 31, so as to enable the nurse or patient attendant to view the patient's non-fluid output.

The chamber 31 is indented just below the filter 36 to receive a drip sensor 40, forming a part of the meter 3. The sensor 40 has a light source 41 located on one side of the chamber 31, just below the lower end of the drip outlet 35, and a light detector 42 located diametrically opposite the source, so that the beam of light from the source is interrupted or attenuated each time a drip of urine falls from the outlet. The output from the sensor 40 is supplied to a battery-powered electronics unit 43, which counts the drops and, from knowledge of the volume of each drop, calculates the volume of urine discharged through the drip outlet 35 in a given time. The electronics unit 43 includes a small display 44 on which is displayed a measure of the volume of urine discharged since the meter was last reset. The electronics unit 43 has two, manually-actuable buttons 45 and 46. One button 45 is a "Reset" button used to reset the volume to zero and the other is a "Total" button 46 used to provide an indication of the total volume of urine discharged over a longer period. The "Total" button 46 can also be used to reset the stored total to zero, such as by holding down the button for a longer time. In this way, for example, the "Total" button may be used to set the total volume to zero at the beginning of the day. Where the clinician or nurse needs to monitor urine flow over a part of the day, such as during a one hour period, he presses the "Reset" button 45, which changes the displayed volume to zero but does not affect the stored volume. The total volume discharged during the day can be displayed at any time by pressing the "Total" button 46.

The electronics unit 43 and the sensor 40 are preferably removable from the chamber 31 so that the chamber can be disposed of and the electronics unit and sensor can be reused with other chambers. In this respect, the electronics unit 43 and sensor 40 may clip onto the outside of the chamber 31. The size and weight of the meter 3 are preferably such that it is self supporting on the inlet 20 of the urine bag 2, without needing any additional support.

Alternative forms of drip sensor could be used, such as, for example, a capacitive sensor.

The urine meter assembly of the present invention can be used on any conventional urine bag or bottle and does not have to be emptied periodically to maintain a measure of volume discharged.

## Claims

1. A urine meter assembly including a urine receptacle (2), a urine tube (1) extending from the patient to the urine receptacle, and a urine meter (3) including a chamber (31) connected intermediate the receptacle (2) and the tube (1), characterised in that the meter (3) includes drip outlet (35) at an upper end of the chamber (31) and connected to receive urine discharged from the tube (1), and an outlet (32) at a lower end of the chamber (31) coupled with an inlet (20) to the urine receptacle (2), and that the meter (3) includes a drip sensor (40) for detecting drips from the drip outlet (35) and a counter (43, 44) for counting the drips and for providing an indication of the quantity of urine having flowed through the meter assembly.

2. A urine meter assembly according to Claim 1, characterised in that the drip sensor (40) is removable from the chamber (31).

3. A urine meter assembly according to Claim 1 or 2, characterised in that the drip sensor (40) includes an optical detector (42).

4. A urine meter assembly according to Claim 3, characterised in that the drip sensor (40) includes a light source (41) located on one side of the chamber (31) and a light detector (42) located on the opposite side of the chamber.

5. A urine meter assembly according to any one of the preceding claims, characterised in that the meter (3) is self supporting.

6. A urine meter assembly according to any one of the preceding claims, characterised in that the meter (3) includes a filter (36) upstream of the drip outlet (35).

7. A urine meter assembly according to any one of the preceding claims, characterised in that the outlet (32) at the lower end of the chamber (31) has a restriction (32') in a lumen through the outlet.

8. A urine meter assembly according to any one of the preceding claims, characterised in that the chamber (31) has a male coupling (34 and 32) at its upper and lower ends.

9. A urine meter assembly according to any one of the preceding claims, characterised in that the urine receptacle is a flexible bag (2).

10. A urine meter assembly according to any one of the preceding claims, characterised in that the counter (43, 44) is switchable to display an indication of the quantity collected over different times.
